# EUROPEAN PATENT SPECIFICATION

(11) **EP 3 897 608 B1**
(45) Date of publication and mention of the grant of the patent: **31.05.2023**
(21) Application number: 19828727.8
(22) Date of filing: 19.12.2019
(51) Int. Cl.: A61K 31/23, A23L 5/00, A61P 37/08, A61K 31/203, A23K 20/158, A23L 33/115, A23L 33/00

(54) **DIETARY BUTYRATE FOR TREATING OR PREVENTING AN ALLERGIC DISORDER**
DIÄTETISCHES BUTYRAT ZUR BEHANDLUNG ODER VORBEUGUNG ALLERGISCHER STÖRUNGEN
BUTYRATE DIÉTÉTIQUE POUR TRAITER OU PREVENIR UN TROUBLE ALLERGIQUE

(30) Priority: 21.12.2018 EP 18215016
(43) Date of publication of application: 27.10.2021
(73) Proprietor: Société des Produits Nestlé S.A., 1800 Vevey (CH)
(72) Inventor: BLANCHARD, Carine, 1052 Le Mont-sur-Lausanne (CH); BOURDEAU, Tristan, 01170 Echevenex (FR); DESTAILLATS, Frederic, 1077 SERVION (CH); FORBES-BLOM, Elizabeth, 1066 EPALINGES (CH); NUTTEN, Sophie, 1607 Palézieux-Village (CH); OERTLING, Heiko, 1012 Lausanne (CH); PATIN, Amaury, 1000 LAUSANNE 26 (CH)
(74) Representative: Elkington and Fife LLP
(86) International application number: PCT/EP2019/086178
(87) International publication number: WO 2020/127642

(56) References cited:
- WO-A1-2017/083069
- MARTIN DIANA ET AL: "In Vitro Intestinal Bioaccessibility of Alkylglycerols Versus Triacylglycerols as Vehicles of Butyric Acid", LIPIDS, vol. 46, no. 3, March 2011 (2011-03), pages 277-285, XP009513752,

## Description

### FIELD OF THE INVENTION

The present invention relates to a dietary source of butyrate having improved organoleptic properties for use in the treatment or prevention of an allergic disorder.

### BACKGROUND TO THE INVENTION

Allergic diseases are now recognized as an epidemic by the World Health Organization (WHO). In Europe and North America, food allergy is estimated to affect nearly 5% of adults and 8% of children (Sicherer, S.H. and Sampson, H.A., 2014. Journal of Allergy and Clinical Immunology, 133(2), pp.291-307). Prevalence of food allergy and anaphylaxis appear to be steadily rising, with the greatest increase observed in infants with food allergies or atopic eczema (Koplin, J.J., et al., 2015. Current opinion in allergy and clinical immunology, 15(5), pp. 409-416).

Alterations in gut microflora composition have been suggested as one explanation for increased incidence of allergy diseases. The gut microbiota promotes regulatory T (Treg) cells that have previously been shown to play a key role in sustaining immune tolerance to allergens (Rivas, M.N. and Chatila, T.A., 2016. Journal of Allergy and Clinical Immunology, 138(3), pp.639-652). In particular, dietary fiber-derived metabolites have been implicated in gut homeostasis and Treg cell biology (Tan, J., et al., 2016. Cell reports, 15(12), pp.2809-2824). Butyric acid is one of the most common short-chain fatty acids (SCFAs) produced by human gut microbiota in response to indigestible dietary fiber in the diet. Salts and esters of butyric acid are known as butyrates or butanoates.

Pre-clinical data has demonstrated that direct oral delivery of butyrate was sufficient to prevent the development of food allergy (Tan, J., et al., 2016. Cell reports, 15(12), pp.2809-2824), and ameliorated allergic lung inflammation (Thio, C.L.P., et al. 2018. Journal of Allergy and Clinical Immunology, 142(6), pp.1867-1883.e12). Additionally, increased fecal butyrate levels were associated with accelerated outgrowth of cow's milk allergy in infants (Canani, R.B., et al., 2016. The ISME journal, 10(3), p.742), and a higher abundance of butyrate-producing gut microbiota were associated with milder symptoms in infants with atopic eczema (Nylund, L., et al., 2015. Allergy, 70(2), pp.241-244). Therefore, butyrate is a strong candidate for modulating food allergic responses by promoting Treg cells.

Beyond the established effect on Treg responses, butyrate has also been demonstrated to affect mast cell activity. Mast cells are thought to exert critical proinflammatory functions, as well as potential immunoregulatory roles, in various immune disorders through the release of mediators such as histamine, leukotrienes, cytokines chemokines, and neutral proteases (Amin, K., 2012. Respiratory medicine, 106(1), pp.9-14). The mouse mast cell line MC/9 exhibits reduced proliferation when exposed to butyrate (Galli, S.J., et al., 1982. The Journal of cell biology, 95(2), pp.435-444), and further studies showed butyrate interferes with mast cell activation, inhibiting mediator release and the production of the proinflammatory cytokine TNFα in mast cells (Diakos, C., et al., 2006. Biochemical and biophysical research communications, 349(2), pp.863-868). Butyrate was also effective at inhibiting mast cell activation and inflammatory mediator production *in vivo* (Wang, C.C., et al., 2018. Innate immunity, 24(1), pp.40-46).

Antigen-specific stimulation of B cells in the presence of butyrate switched plasma cell differentiation to regulatory B cell (Breg) development and IL-10 production, and Breg induction *in vivo* was associated with a concomitant reduction in intestinal mast cell numbers following an experimental food allergy (Shi, Y., et al., 2015. Scientific reports, 5, p.17651). Butyrate may also affect mast cell responses via impacting innate lymphoid cell type 2 (ILC2) activation and/or cytokine response. Butyrate, but not acetate or propionate was sufficient to ameliorate ILC2 driven allergic inflammation (including eosinophilic inflammation), and reduced cytokine production in human ILC2 cells (Thio, C.L.P., et al. 2018. Journal of Allergy and Clinical Immunology, 142(6), pp.1867-1883.e12). ILC2 responses have been implicated in promoting IgE-mast cell mediated experimental food allergy (Chen, C.Y., et al., 2015. Immunity, 43(4), pp.788-802).

Common sources of butyrate are butyric acid and tributyrin, a triglyceride made of three ester functional groups with three butyrate moieties and the glycerol backbone. Butyric acid and tributyrin are both food additives that are generally regarded as safe (GRAS) (21CFR582.60 and 21CFR184.1903 respectively), and are natural components of many dairy items. However, butyric acid is associated with negative sensory qualities such as vomit-like, fecal, and cheesy aroma attributes. Tributyrin also has negative sensory qualities, in particular high bitterness. These unpleasant taste and odor attributes can make the oral administration of compositions including these compounds particularly difficult, especially in the pediatric population. Butyrate components from dairy cannot be enriched and thus significant volumes of dairy fat would need to be consumed which is not feasible for practical and nutritional reasons, not least as it would lead to large amount of unwanted calorie derived from animal fat.

WO 2017/083069 A1 discloses reducing the incidence of allergy and/or improving tolerance to cow's milk allergy in target subjects with butyrate. The dietary butyrate may be provided by dairy lipids and/ or triglyceride bound forms of butyrate.

Accordingly, it would be beneficial to provide a food-grade source of butyrate having improved organoleptic properties as compared to available solutions for use in the treatment or prevention of an allergic disorder.

### SUMMARY OF THE INVENTION

The present invention relates to compounds that are a source of butyrate having improved organoleptic properties for use in the treatment or prevention of an allergic disorder. In particular, the compounds have improved odor and/or taste relative to butyric acid, butyrate salts and/or tributyrin. The compounds may be used as a dietary source of butyric acid. The compounds may be used in, for example, nutritional compositions, dietary supplements, infant formulas, follow-on formulas, beverages and pet care products.

The scope of the present invention is defined in the claims; any other disclosure in the present description is provided for referential purposes, only.

Any references in the description to methods of treatment refer to the compounds, pharmaceutical compositions and medicaments of the present invention for use in a method for treatment of the human (or animal) body by therapy (or for diagnosis).

According to a first aspect of the present invention there is provided a compound having the formula 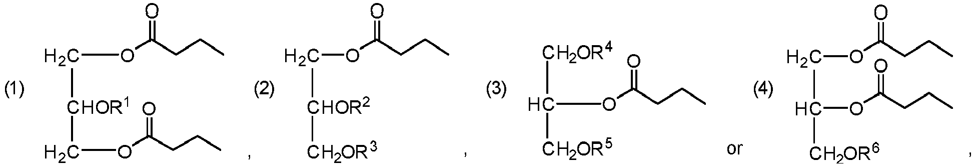 or combinations thereof, for use in the treatment or prevention of an allergic disorder, wherein R¹, R², R³, R⁴, R⁵ and R⁶ are independently, a long chain fatty acid having between 16 and 20 carbons.

According to another aspect of the present invention there is provided a composition comprising a compound having the formula (1), (2), (3) or (4) or combinations thereof, for use in the treatment or prevention of an allergic disorder, wherein R¹, R², R³, R⁴, R⁵ and R⁶ are independently, a long chain fatty acid having between 16 and 20 carbons.

In one embodiment the composition comprises the compound having formula (1), the compound having formula (2), the compound having formula (3) and the compound having formula (4).

The composition may comprise the compound having formula (1) and the compound having formula (2).

The composition may comprise the compound having formula (1) and the compound having formula (3).

The composition may comprise the compound having formula (1) and the compound having formula (4).

The composition may comprise the compound having formula (2) and the compound having formula (3).

The composition may comprise the compound having formula (2) and the compound having formula (4).

The composition may comprise the compound having formula (3) and the compound having formula (4).

The composition may comprise the compound having formula (1) the compound having formula (2), and the compound having formula (3).

The composition may comprise the compound having formula (1) the compound having formula (2), and the compound having formula (4).

The composition may comprise the compound having formula (1) the compound having formula (3), and the compound having formula (4).

The composition may comprise the compound having formula (2) the compound having formula (3), and the compound having formula (4).

The composition may comprise the compound having formula (1), the compound having formula (2), the compound having formula (3) and the compound having formula (4).

In one embodiment the compounds having formula (1), (2), (3) and (4), comprise at least 50%, 60%, 70%, 80%, 90%, 95% or 99% by weight of the total triglycerides of the composition.

In one embodiment the compounds having formula (1), (2), (3) and (4), comprise at least 50%, 60%, 70%, 80%, 90%, 95% or 99% by weight of the total butyrate moiety containing triglycerides in the composition.

In one embodiment, the compound having the formula (4) is the main butyrate moiety containing triglyceride in the composition.

In one embodiment, the composition comprises the compound of formula (1) and the compound of formula (4), and the combination of the compound having formula (1) and the compound having the formula (4) is present in an amount of at least 30%, 40%, 50%, 60%, 70%, 80%, or 90% by weight of the total butyrate moiety containing triglycerides in the composition.

In one embodiment tributyrin comprises less than 10% by weight of the total triglycerides in the composition, preferably less than 8% by weight, more preferably less than 5% by weight of the total triglycerides in the composition.

In one embodiment the composition further comprises vitamin A and/or dietary fiber and/or probiotic.

In one embodiment the composition is a nutritional composition, preferably wherein the nutritional composition is a dietary supplement, an infant formula, a follow on-formula, a beverage or a pet care product.

In one embodiment R¹, R², R³, R⁴, R⁵ and/or R⁶ is an unsaturated fatty acid, preferably monounsaturated.

In one embodiment R¹, R², R³, R⁴, R⁵ and/or R⁶ is selected from the group consisting of oleic acid, palmitic acid, or linoleic acid, preferably each of R¹, R², R³, R⁴, R⁵ and R⁶ is oleic acid.

In one embodiment the allergic disorder is selected from one or more of the group consisting of: a food allergy, a food intolerance, a respiratory allergy and a skin allergy.

In one embodiment the allergic disorder is selected from one or more of the group consisting of: rhinitis, asthma, hives or hay fever, allergic conjunctivitis, dermatitis, atopic dermatitis, contact dermatitis, eczema, atopic eczema, urticaria, psoriasis, eosinophilic oesophagitis and an eosinophilic-associated gastrointestinal disease, allergic diarrhea, vomiting, abdominal pain or bloating.

In one embodiment the allergen in the allergic disorder is selected from one or more of: a food allergen, dust mite, pollen, molds or mold spores, weed pollen, tree pollen, grass pollen, fleas, pet or animal hair, feathers or pet dander.

In one embodiment the allergen in the allergic disorder is a food allergen, preferably wherein the food allergen is selected from: a nut, tree nut, peanut, fish, shellfish, molluscs, crustaceans, milk, egg, soy, gluten, cereals, wheat, oats, barley, rye, celery, corn, lupin, sulphites, sesame, mustard, rice, poultry and meat.

In one embodiment the compounds or combinations thereof have improved organoleptic properties relative to butyric acid, tributyrin and/or butyrate salts.

In one embodiment the compounds is provided to a young or adult mammal, preferably, an infant a toddlers, a young or adult human, a pet or a farm animal.

### BRIEF DESCRIPTION OF THE FIGURES

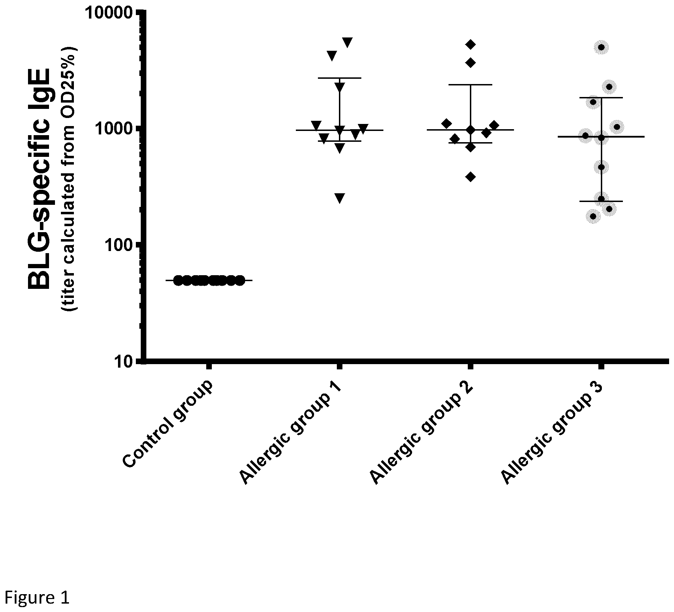

**Figure 1** is a graphical representation of BLG-specific Immunoglobulin E (IgE) response in milk protein β-lactoglobulin (BLG) sensitized and control group mice.

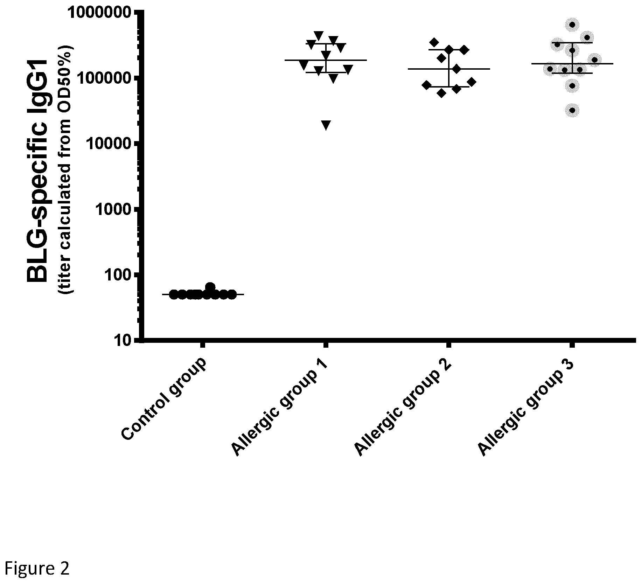

**Figure 2** is a graphical representation of BLG-specific Immunoglobulin G1 (!gG1) response in milk protein β-lactoglobulin (BLG) sensitized and control group mice.

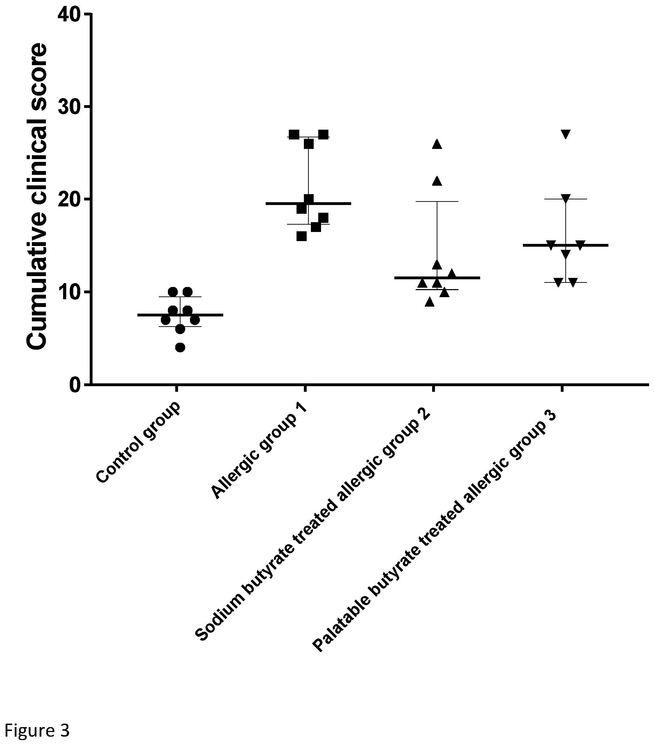

**Figure 3** is a graphical representation of cumulative clinical score following oral OVA challenge in mice "control group" (negative control), "Allergic group 1" (BLG sensitized positive control); "Sodium butyrate treated allergic group 2" and "Palatable butyrate treated allergic group 3".

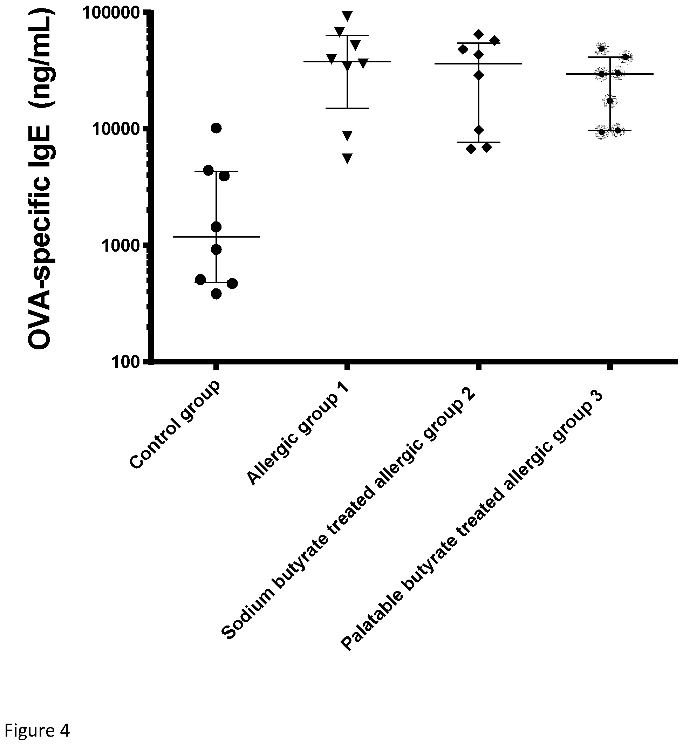

**Figure 4** is a graphical representation of OVA-specific IgE response on oral OVA challenge in allergic and control group mice, "control group" (negative control), "Allergic group 1" (BLG sensitized positive control); "Sodium butyrate treated allergic group 2" and "Palatable butyrate treated allergic group 3".

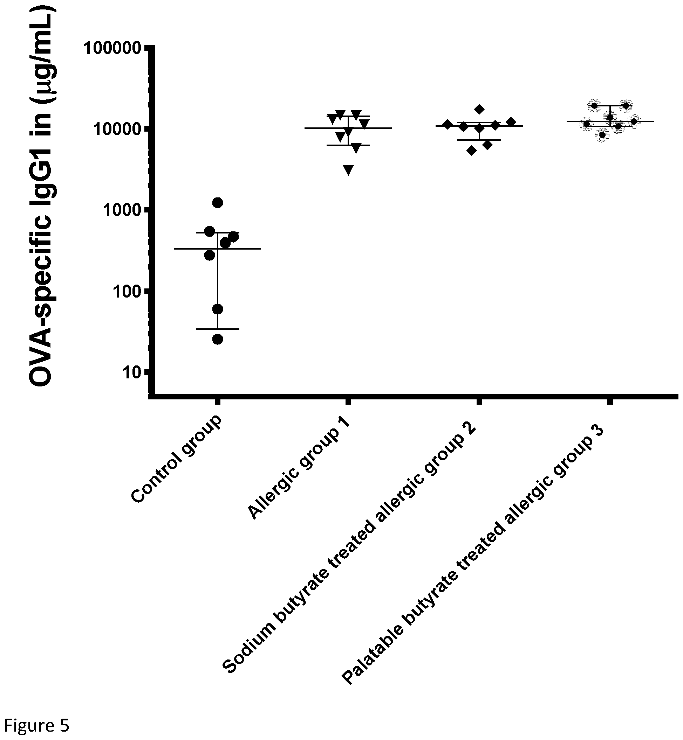

**Figure 5** is a graphical representation of OVA-specific IgG1 response on oral OVA challenge in allergic and control group mice, "control group" (negative control), "Allergic group 1" (BLG sensitized positive control); "Sodium butyrate treated allergic group 2" and "Palatable butyrate treated allergic group 3".

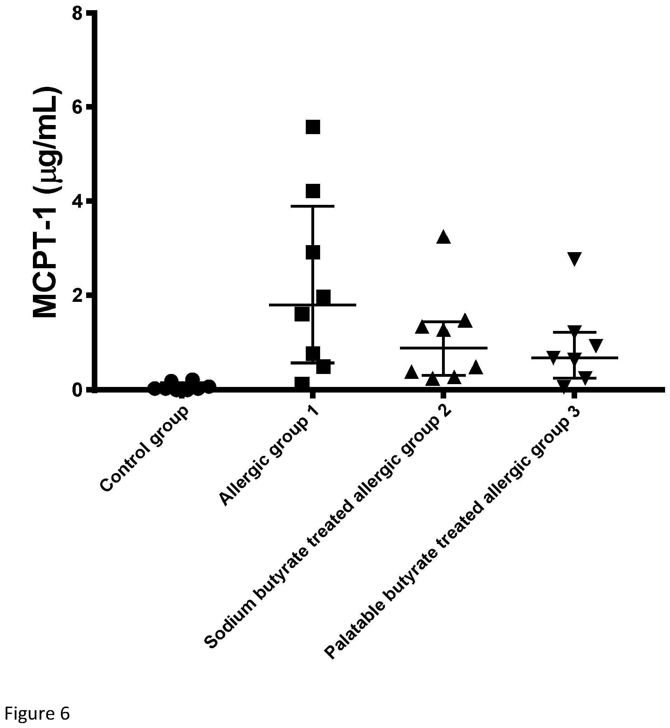

**Figure 6** is a graphical representation of mast cell protease-1 (MCPT-1) on oral OVA challenge in allergic and control group mice, "control group" (negative control), "Allergic group 1" (BLG sensitized positive control); "Sodium butyrate treated allergic group 2" and "Palatable butyrate treated allergic group 3".

### DETAILED DESCRIPTION OF THE INVENTION

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of".

### Triglycerides

A triglyceride (also known as a triacylglycerol) is a triester that is derived from glycerol and three fatty acids. Under hydrolysis conditions such as those during digestion, triglycerides may be a source of fatty acids. For instance, tributyrin is potentially a source of three moles of butyric acid per mole of tributyrin.

Fatty acids are carboxylic acids with a long tail (chain). Fatty acids may be either unsaturated or saturated. Fatty acids which are not attached to other molecules are referred to as free fatty acids (FFA).

The term "fatty acid moiety" refers to the part of the triglyceride that originates from a fatty acid in an esterification reaction with glycerol. The triglycerides used in the present invention comprise at least one butyric acid moiety and at least one long chain fatty acid moiety.

Preferred long chain fatty acids for use in the present invention are fatty acids that have 16 to 20 carbon atoms. Examples of long chain fatty acid include oleic acid, palmitic acid, stearic acid and linoleic acid. Preferably, the long chain fatty acid is oleic acid. For example, the present invention provides a compound having the formula 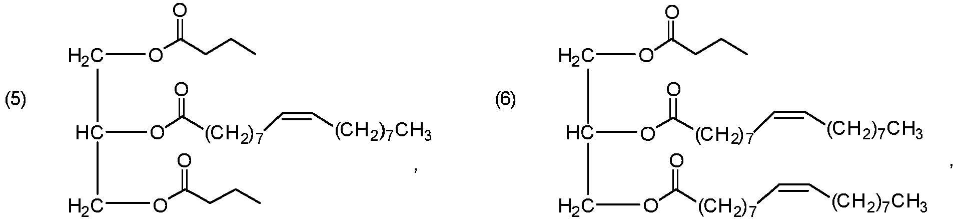 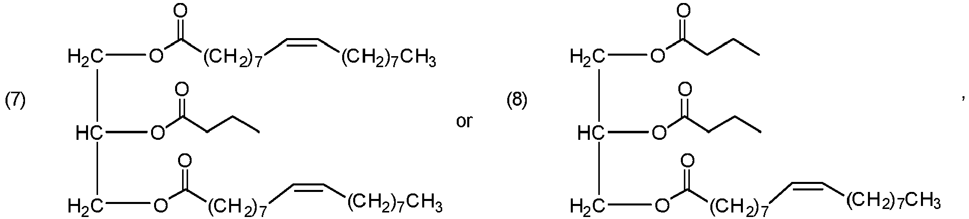 or combinations thereof, for use in the treatment or prevention of an allergic disorder.

Other examples of triglycerides which may be used in the present invention include: 1,3-dibutyryl-2-linoleoylglycerol, 1,3-dibutyryl-2-stearoylglycerol, 1-butyryl-2-oleoyl-3-palmitoylglycerol, 1-palmitoyl-2-oleoyl-3-butyrylglycerol,1-butyryl-2-oleoyl-3-linoleoylglycerol, 1-linoleoyl-2-oleoyl-3-butyrylglycerol, 1-oleoyl-2-butyryl-3-linoleoylglycerol, 1-linoleoyl-2-butyryl-3-oleoylglycerol, 1-butyryl-2-linoleoyl-3-oleoylglycerol, 1-oleoyl-2-linoleoyl-3-butyrylglycerol, 1-butyryl-2-stearoyl-3-oleoylglycerol, 1-oleoyl-2-stearoyl-3-butyrylglycerol, 1-butyryl-2-oleoyl-3-stearoylglycerol, 1-stearoyl-2-oleoyl-3-butyrylglycerol, 1,2-dioleoyl-3-palmitoylglycerol, 1-palmitoyl-2,3-dioleoylglycerol, 1,2-dioleoyl-3-linoleoylglycerol and 1-linoleoyl-2,3-dioleoylglycerol.

The triglycerides of the present invention may be synthesised by, for example, esterification of long chain fatty acid(s) and butyric acid with glycerol.

The triglycerides of the present invention may be synthesised by, for example, interesterification between tributyrin and another triglyceride containing long chain fatty acids.

In one embodiment, high oleic sunflower oil is the source of the long chain fatty acids. This generates triglycerides containing predominantly butyrate and oleate moieties. The compounds are dairy-free, cholesterol-free and vegan. Fatty acids are liberated from triglycerides due to lipases, naturally present in the gastrointestinal tract. Relative to butyrate salts, the compounds do not add additional mineral salts to the final formulation.

Alternative methods of triglyceride synthesis can be routinely determined by a person skilled in the art.

By way of example, a method of obtaining 1,3-dibutyryl-2-palmitoylglycerol (BPB) is shown below:

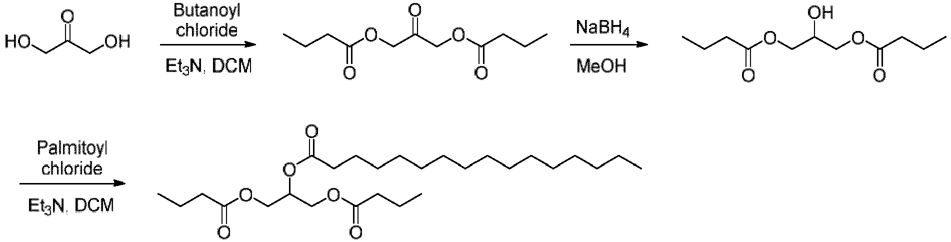

As another example, the triglycerides may be synthesized by esterification of a long chain fatty acid monoacylglycerol (MAG) with butyric acid (BA).

For example, by esterification of long chain fatty acid monoacylglycerol (MAG) with butyric acid (BA) with the removal of water. By way of example a method of obtaining 1,2-dibutyryl-3-oleoylglycerol is shown below:

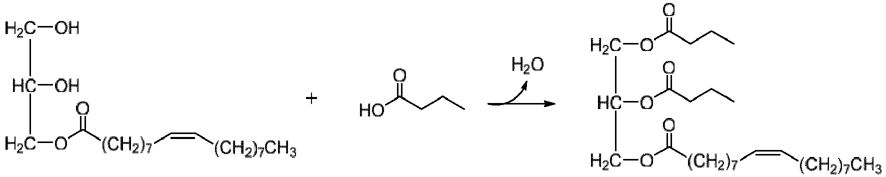

The esterification reaction is preferably carried out with butyric acid (BA): monoacylglycerol (MAG) molar ratio of ≥ 2; i.e. in a molar excess of butyric acid. Removal of water can be carried by conventional methods, routinely used in the art.

A single butyrate moiety containing triglyceride may be used herein. Alternatively, a mixture of different butyrate moiety containing triglycerides may be used.

The triglycerides may be further subjected to decolouration and/or deodorization steps conventional in the art and well known to the person skilled in the art. For example, as conventionally used in the manufacture of vegetable oils.

### Compositions

Compounds of the present invention may be administered in the form of a composition. Thus, the present invention provides compositions comprising butyrate moiety containing triglycerides referred to herein, for use in the treatment or prevention of an allergic disorder.

In one embodiment, a combination of a compound having formula (1) and a compound having formula (2) is present in the composition as defined herein.

In one embodiment the compound having formula (1) is present in an amount of at least 10% by weight of the total triglycerides in the composition, and the compound having formula (2) is present in an amount of at least 10% by weight of the total triglycerides in the composition.

In one embodiment the compound having formula (1) is present in an amount of at least 15% by weight of the total triglycerides in the composition, and the compound having formula (2) is present in an amount of at least 15% by weight of the total triglycerides in the composition.

In one embodiment the compound having formula (1) is present in an amount of at least 20% by weight of the total triglycerides in the composition, and the compound having formula (2) is present in an amount of at least 20% by weight of the total triglycerides in the composition.

In one embodiment the compound having formula (1) is present in an amount of at least 20% by weight of the total triglycerides in the composition, and the compound having formula (2) is present in an amount of at least 30% by weight of the total triglycerides in the composition.

In one embodiment the compound having formula (1) comprises about 20% to about 40% by weight of the total triglycerides in the composition, and/or the compound having formula (2) comprises about 30% to about 40% by weight of the total triglycerides in the composition.

In one embodiment the compound having formula (1) and the compound having formula (2) comprise at least 20%, 30%, 40%, 50%, 60% or 70% by weight of the total triglycerides in the composition, preferably about 40% to about 80%, or about 50% to about 75% by weight of the total triglycerides in the composition.

In one embodiment the composition further comprises the compound having formula (3), preferably wherein the compound having formula (3) comprises at least 2%, 3%, 4% or 5% by weight of the total triglycerides in the composition, and/or the composition further comprises the compound having formula (4), preferably wherein the compound having formula (4) comprises at least 1%, 2% or 3% by weight of the total triglycerides in the composition.

In one embodiment the compound having formula (1) is present in an amount of at least 20% by weight of the total butyric acid containing triglycerides in the composition, and the compound having formula (2) is present in an amount of at least 30% by weight of the total butyric acid containing triglycerides in the composition.

In one embodiment the compound having formula (1) comprises about 30% to about 50% by weight of the total butyric acid containing triglycerides in the composition, and/or the compound having formula (2) comprises about 40% to about 60% by weight of the total butyric acid containing triglycerides in the composition.

In one embodiment the compound having formula (1) and the compound having formula (2) comprise at least 20%, 30%, 40%, 50%, 60%, 70% or 80% by weight of the total butyric acid containing triglycerides in the composition, preferably about 60% to about 90% by weight of the total butyric acid containing triglycerides in the composition.

In one embodiment, the compound having the formula (4) is the main butyrate moiety containing triglyceride in the composition.

In one embodiment, the composition comprises the compound of formula (1) and the compound of formula (4), and the combination of the compound having formula (1) and the compound having the formula (4) is present in an amount of at least 30%, 40%, 50%, 60%, 70%, 80%, or 90% by weight of the total butyrate moiety containing triglycerides in the composition.

In one embodiment the compound having formula (5) comprises at least 10% by weight of the total triglycerides in the composition, and/or the compound having formula (6) comprises at least 10% by weight of the total triglycerides in the composition.

In one embodiment the compound having formula (5) comprises at least 15% by weight of the total triglycerides in the composition, and/or the compound having formula (6) comprises at least 15% by weight of the total triglycerides in the composition.

In one embodiment the compound having formula (5) comprises at least 15% by weight of the total triglycerides in the composition, and/or the compound having formula (6) comprises at least 20% by weight of the total triglycerides in the composition.

In one embodiment the compound having formula (5) comprises at least 20% by weight of the total triglycerides in the composition, and/or the compound having formula (6) comprises at least 20% by weight of the total triglycerides in the composition.

In one embodiment the compound having formula (5) comprises about 15% to about 30% by weight of the total triglycerides in the composition, and/or the compound having formula (6) comprises about 20% to about 30% by weight of the total triglycerides in the composition.

In one embodiment the compound having formula (5) and the compound having formula (6) comprise at least 20%, 30% or 40% by weight of the total triglycerides in the composition, preferably about 30% to about 60%, or about 40% to about 50% by weight of the total triglycerides in the composition.

In one embodiment the composition further comprises the compound having formula (7), preferably wherein the compound having formula (7) comprises at least 2% or 3% by weight of the total triglycerides in the composition, and/or the composition further comprises the compound having formula (8), preferably wherein the compound having formula (8) comprises at least 2% or 3% by weight of the total triglycerides in the composition.

In one embodiment the compound having formula (5) comprises at least 10% by weight of the total butyrate moiety containing triglycerides in the composition, and the compound having formula (6) comprises at least 10% by weight of the total butyrate moiety containing triglycerides in the composition.

In one embodiment the compound having formula (5) comprises at least 15% by weight of the total butyrate moiety containing triglycerides in the composition, and the compound having formula (6) comprises at least 15% by weight of the total butyrate moiety containing triglycerides in the composition.

In one embodiment the compound having formula (5) comprises at least 15% by weight of the total butyrate moiety containing triglycerides in the composition, and the compound having formula (6) comprises at least 20% by weight of the total butyrate moiety containing triglycerides in the composition.

In one embodiment the compound having formula (5) comprises at least 20% by weight of the total butyrate moiety containing triglycerides in the composition, and the compound having formula (6) comprises at least 20% by weight of the total butyrate moiety containing triglycerides in the composition.

In one embodiment the compound having formula (5) comprises about 15% to about 30% by weight of the total butyrate moiety containing triglycerides in the composition, and the compound having formula (6) comprises about 20% to about 30% by weight of the total butyrate moiety containing triglycerides in the composition.

In one embodiment the composition further comprises the compound having formula (7), preferably wherein the compound having formula (7) comprises at least 2% or 3% by weight of the total butyrate moiety containing triglycerides in the composition, and/or the composition further comprises the compound having formula (8), preferably wherein the compound having formula (8) comprises at least 2% or 3% by weight of the total butyrate moiety containing triglycerides in the composition.

In another embodiment, the compound having the formula (8) is the main butyrate moiety containing triglyceride in the composition.

In one embodiment, the compound of formula (8) comprises at least 20%, at least 30%, at least 40%, at least 50%, or at least 60%, at least 70%, at least 80% or at least 90%, by weight of the total butyrate moiety containing triglycerides in the composition.

In one embodiment the compound having formula (8) comprises about 20% to about 95% by weight of the total butyrate moiety containing triglycerides in the composition, for example about 30% to about 90%, or about 40% to about 80% by weight of the total butyrate moiety containing triglycerides in the composition, for example about 50% to about 70% by weight of the total butyrate moiety containing triglycerides in the composition.

In one embodiment the compound having formula (8) comprises about 50% to about 90% by weight of the total butyrate moiety containing triglycerides in the composition, for example about 60% to about 80% by weight of the total butyrate moiety containing triglycerides in the composition.

In one embodiment, the composition comprises the compound of formula (8) and the compound of formula (5), and the combination of the compound having formula (8) and the compound having the formula (5) is present in an amount of at least 30%, 40%, 50%, 60%, 70%, 80%, or 90% by weight of the total butyrate moiety containing triglycerides in the composition.

In one embodiment composition of the present invention may further comprise 1,3-dibutyryl-2-linoleoylglycerol, 1,3-dibutyryl-2-stearoylglycerol, 1-butyryl-2-oleoyl-3-palmitoylglycerol, 1-palmitoyl-2-oleoyl-3-butyrylglycerol,1-butyryl-2-oleoyl-3-linoleoylglycerol, 1-linoleoyl-2-oleoyl-3-butyrylglycerol, 1-oleoyl-2-butyryl-3-linoleoylglycerol, 1-linoleoyl-2-butyryl-3-oleoylglycerol, 1-butyryl-2-linoleoyl-3-oleoylglycerol, 1-oleoyl-2-linoleoyl-3-butyrylglycerol, 1-butyryl-2-stearoyl-3-oleoylglycerol, 1-oleoyl-2-stearoyl-3-butyrylglycerol, 1-butyryl-2-oleoyl-3-stearoylglycerol, 1-stearoyl-2-oleoyl-3-butyrylglycerol, 1,2-dioleoyl-3-palmitoylglycerol, 1-palmitoyl-2,3-dioleoylglycerol, 1,2-dioleoyl-3-linoleoylglycerol and/or 1-linoleoyl-2,3-dioleoylglycerol.

In one embodiment, tributyrin comprises less than 10% by weight of the total butyrate moiety containing triglycerides in the composition, preferably less than 8% by weight, more preferably less than 5% by weight of the total butyrate moiety containing triglycerides in the composition.

The composition of the present invention can be in, for example, a solid (e.g. powder), liquid or gelatinous form.

The composition of the present invention can be in, for example, tablet, dragee, capsule, gel cap, powder, granule, solution, emulsion, suspension, coated particle, spray-dried particle or pill.

The composition may in the form of a pharmaceutical composition and may comprise one or more suitable pharmaceutically acceptable carriers, diluents and/or excipients. Examples of such suitable excipients for compositions described herein may be found in the "Handbook of Pharmaceutical Excipients", 2nd Edition, (1994), Edited by A Wade and PJ Weller. Acceptable carriers or diluents for therapeutic use are also well known in the pharmaceutical art, and are described, for example, in Remington's Pharmaceutical Sciences, Mack Publishing Co. (A. R. Gennaro edit. 1985).

The pharmaceutical compositions may comprise as, or in addition to, the carrier, excipient or diluent any suitable binder(s), lubricant(s), suspending agent(s), coating agent(s) and/or solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like.

Preservatives, stabilisers, dyes and even flavouring agents may be provided in the composition. Examples of preservatives include sodium benzoate, sorbic acid and esters of p-hydroxybenzoic acid. Antioxidants and suspending agents may be also used.

The composition may be a nutritional composition.

The expression "nutritional composition" means a composition that nourishes a subject. This nutritional composition is preferably taken orally, and it may include a lipid or fat source and a protein source. It may also contain a carbohydrate source. In one embodiment, the nutritional composition contains only a lipid or fat source. In other specific embodiments, the nutritional composition contains a lipid (or fat) source with a protein source, a carbohydrate source or both.

In some specific embodiments, the nutritional composition according to the invention is an "enteral nutritional composition" that is to say a foodstuff that involves the gastrointestinal tract for its administration. The gastric introduction may involve the use of a tube through the oro/nasal passage or a tube in the belly leading directly to the stomach. This may be used especially in hospitals or clinics.

The composition according to the invention can be a dietary supplement.

The term "dietary supplement" may be used to complement the nutrition of an individual (it is typically used as such but it might also be added to any kind of compositions intended to be ingested). It may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The dietary supplement may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

When the composition is a supplement, it can be provided in the form of unit doses.

The composition according to the invention can be an infant formula (e.g. a starter infant formula), a follow-up or follow-on formula, a growing-up milk, a baby food, an infant cereal composition, or a fortifier such as a human milk fortifier.

The expression "infant formula" as used herein refers to a foodstuff intended for particular nutritional use by infants during the first months of life and satisfying by itself the nutritional requirements of this category of person (e.g., Article 2(c) of the European Commission Directive 91/321/EEC 2006/141/EC of 22 December 2006 on infant formulae and follow-on formulae).

Generally a starter formula is for infants from birth as breast-milk substitute. A follow-up or follow-on formula is given from the sixth month onwards. It constitutes the principal liquid element in the progressively diversified diet of this category of person. The "growing-up milks" (or GUMs) are given from one year onwards. It is generally a milk-based beverage adapted for the specific nutritional needs of young children.

The term "fortifier" refers to liquid or solid nutritional compositions suitable for mixing with breast milk (human milk) or infant formula. The term "breast milk" should be understood as the mother's milk or the colostrum of the mother or a donor's milk or the colostrum of a donor's milk.

The composition according to the invention can be a dairy product, a liquid beverage, a beverage powder, a dehydrated soup, a dietary supplement, a meal replacement, a nutritional bar, a cereal, a confectionery product or a dry pet food.

The composition may further comprise dietary fiber. Dietary fiber has been shown to enhance oral tolerance and protect against food allergy (Tan, J., et al., 2016. Cell reports, 15(12), pp.2809-2824). The "dietary fiber" may comprise at least one non-digestible oligosaccharide (e.g. prebiotics). The prebiotics may be present in an amount between 0.3 and 10% by weight of composition. Dietary fiber and/or prebiotics may promote the production of endogenous butyrate by gut microflora and thus provide additional beneficial effects.

Prebiotics are usually non-digestible in the sense that they are not broken down and absorbed in the stomach or small intestine and thus remain intact when they pass into the colon where they are selectively fermented by the beneficial bacteria. Examples of prebiotics include certain oligosaccharides, such as fructooligosaccharides (FOS), inulin, xylooligosaccharides (XOS), polydextrose or any mixture thereof. In a particular embodiment, the prebiotics may be fructooligosaccharides and/or inulin. In a specific embodiment, the prebiotics is a combination of FOS with inulin such as in the product sold by BENEO-Orafti under the trademark Orafti^{®} oligofructose (previously Raftilose^{®}) or in the product sold by BENEO-Orafti under the trademark Orafti^{®} inulin (previously Raftiline^{®}). Another example is a combination of 70% short chain fructooligosaccharides and 30% inulin, which is registered by Nestle under the trademark "Prebio 1". The nutritional composition of the invention can also comprise at least one milk's oligosaccharide that can be a BMO (bovine milk oligosaccharide) and/or a HMO (human milk oligosaccharide). In a particular embodiment, the nutritional composition according to the invention comprises an oligosaccharide mixture comprising from 0.1 to 4.0 wt% of N-acetylated oligosaccharide(s), from 92.0 to 98.5 wt% of the galacto-oligosaccharide(s) and from 0.3 to 4.0 wt% of sialylated oligosaccharide(s).

The composition of the present invention can further comprise at least one probiotic (or probiotic strain), such as a probiotic bacterial strain. Consumption of probiotic strains may also promote the production of endogenous butyrate by gut microflora and thus provide additional beneficial effects.

The probiotic microorganisms most commonly used are principally bacteria and yeasts of the following genera: Lactobacillus spp., Streptococcus spp., Enterococcus spp., Bifidobacterium spp. and Saccharomyces spp.

In some particular embodiments, the probiotic is a probiotic bacterial strain. In some specific embodiments, it is Bifidobacteria and/or Lactobacilli.

The nutritional composition according to the invention may contain from 10e3 to 10e12 cfu of probiotic strain, more preferably between 10e7 and 10e12 cfu such as between 10e8 and 10e10 cfu of probiotic strain per g of composition on a dry weight basis.

In one embodiment the probiotics are viable. In another embodiment the probiotics are non-replicating or inactivated. It may also be probiotic parts such as cell wall components or products of the probiotic metabolism. There may be both viable probiotics and inactivated probiotics in some other embodiments. The nutritional composition of the invention can further comprise at least one phage (bacteriophage) or a mixture of phages, preferably directed against pathogenic Streptococci, Haemophilus, Moraxella and Staphylococci.

The nutritional composition of the invention, and especially the infant formula, generally contains a protein source, a carbohydrate source and a lipid source. In some embodiments however, especially if the nutritional composition of the invention is a supplement or a fortifier, there may be only lipids (or a lipid source).

The nutritional composition according to the invention may contain a protein source. The protein may be in an amount of from 1.6 to 3 g per 100 kcal. In some embodiments, especially when the composition is intended for preterm infants/young children, the protein amount can be between 2.4 and 4 g/100kcal or more than 3.6 g/100kcal. In some other embodiments the protein amount can be below 2.0 g per 100 kcal, e.g. between 1.8 to 2 g/100kcal, or in an amount below 1.8g per 100 kcal.

Protein sources based on, for example, whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in any desired proportions. In some embodiments the protein source is whey predominant (i.e. more than 50% of proteins are coming from whey proteins, such as 60%> or 70%>). The proteins may be intact or hydrolysed or a mixture of intact and hydrolysed proteins. In some embodiments, the protein source may also be provided partially or entirely in the form of added amino acids.

By the term "intact" is meant that the main part of the proteins are intact, i.e. the molecular structure is not altered, for example at least 80% of the proteins are not altered, such as at least 85% of the proteins are not altered, preferably at least 90% of the proteins are not altered, even more preferably at least 95% of the proteins are not altered, such as at least 98% of the proteins are not altered. In a particular embodiment, 100% of the proteins are not altered.

The term "hydrolysed" means in the context of the present invention a protein, which has been hydrolysed or broken down into its component amino acids.

The proteins may be either fully or partially hydrolysed. If hydrolysed proteins are required, the hydrolysis process may be carried out as desired and as is known in the art. For example, whey protein hydrolysates may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10%> by weight of lysine; for example about 7% by weight of lysine which greatly improves the nutritional quality of the protein source.

In one particular embodiment the proteins of the composition are hydrolysed, extensively hydrolysed or partially hydrolysed. The degree of hydrolysis (DH) of the protein can be between 2 and 20, or between 8 and 40, or between 20 and 60 or between 20 and 80 or more than 10, 20, 40, 60, 80 or 90. For example, nutritional compositions containing hydrolysates having an extent of hydrolysis less than about 15% are commercially available from Nestle Company under the trade mark Peptamen^{®}.

In some embodiments the protein is extensively hydrolysed. For example infant formula products, containing extensively hydrolyzed protein are commercially available from Nestle Company under the trade mark Althera^{®}, Alfaré^{®}.

At least 70%, 80%, 85%, 90%, 95% or 97% of the proteins may be hydrolysed. In a particular embodiment, 100% of the proteins are hydrolysed.

In one particular embodiment the proteins are provided as amino acids. For example infant formula products based o amino acids as the protein source are commercially available from Nestle Company under the trade mark Alfamino^{®}.

In one particular embodiment the proteins of the composition are plant based protein.

The nutritional composition according to the present invention may contain a carbohydrate source. This is particularly preferable in the case where the nutritional composition of the invention is an infant formula. In this case, any carbohydrate source conventionally found in infant formulae such as lactose, sucrose, saccharose, maltodextrin, starch and mixtures thereof may be used although one of the preferred sources of carbohydrates for infant formula is lactose.

The nutritional composition of the invention may also contain all vitamins and minerals understood to be essential in the daily diet and in nutritionally significant amounts. Minimum requirements have been established for certain vitamins and minerals. Examples of minerals, vitamins and other nutrients optionally present in the composition of the invention include vitamin A, vitamin B1, vitamin B2, vitamin B3, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary depending on the intended population.

In one particular embodiment the composition may further comprise vitamin A and/or retinol. Butyrate may promote CD103-expressing dendritic cells (CD103+ DCs) to convert vitamin A to retinoic acid, which promotes the differentiation of naive T cells into Treg cells (Tan, J., et al., 2016. Cell reports, 15(12), pp.2809-2824). In particular, Tan et al. have shown that dietary fiber with vitamin A increases the potency of tolerogenic CD103+ DCs.

If necessary, the nutritional composition of the invention may contain emulsifiers and stabilisers such as soy, lecithin, citric acid esters of mono- and diglycerides, and the like. The nutritional composition of the invention may also contain other substances which may have a beneficial effect such as lactoferrin, osteopontin, TGFbeta, slgA, glutamine, nucleotides, nucleosides, and the like.

The nutritional composition according to the invention may be prepared in any suitable manner. For example, a composition may be prepared by blending together the components in appropriate portions, optionally blended with one or more carriers and then mixing the dry blended mixture with a liquefier to form a liquid mixture. The liquid mixture may then be homogenised, pasteurised and optionally spray-dried if the final product is to be a powder. The composition may be homogenised before pasteurisation or after pasteurisation.

For example, a formula such as an infant formula may be prepared by blending together the protein source, the carbohydrate source and the fat source, in appropriate proportions. If used, the emulsifiers may be included at this point. The vitamins and minerals may be added at this point but they are usually added later to avoid thermal degradation. Any lipophilic vitamins, emulsifiers and the like may be dissolved into the fat source prior to blending. Water, preferably water that has been subjected to reverse osmosis, may then be mixed in to form a liquid mixture. The temperature of the water is conveniently in the range between about 50°C and about 80°C to aid dispersal of the ingredients. Commercially available liquefiers may be used to form the liquid mixture. Any oligosaccharides may be added at this stage, especially if the final product is to have a liquid form. If the final product is to be a powder, they may likewise be added at this stage if desired. The liquid mixture is then homogenised, for example in two stages.

### Allergic Disorders

The compounds defined herein are a source of butyrate/butyric acid and may therefore be used for preventing or treating allergic disorders. The compounds may be used in infants, children or adults.

Allergic sensitization in childhood, especially in early childhood and especially to food allergens, is critical and of highest interest as development of an "allergic phenotype" or "atopy" has been shown to facilitate subsequent sensitization to other allergens. Eczema in infants is also a predisposing factor for a later development of food allergy or other type of allergies. Hence allergies in childhood can be the first step of an allergic cascade leading to multiple allergies later in life, a process commonly referred to as the "Atopic March". For example, children with persistent food hypersensitivity early in life have a dramatically increased risk to develop allergic rhinitis (hay fever) or asthma later in childhood (Ostblom, E. et al. (2008); Phenotypes of food hypersensitivity and development of allergic diseases during the first 8 years of life, Clinical and Experimental Allergy, 38 (8): 1325-1332). Children with milder forms of food hypersensitivity also have increased risk for development of respiratory allergies but to a lesser degree than children with persistent food hypersensitivity. Therefore, attenuating the severity of food hypersensitivity may be crucial for slowing down the "Atopic March". In this context the management of allergic episodes and prevention of allergies are, in childhood and infancy, of the highest importance.

The immune system of infants is actively developing all along the few first years of life. Acting on, preventing, avoiding, managing, reducing or modulating the allergic reactions in such young patients can influence their allergic profile short term but also longer term for later in life. In one embodiment the prevention or treatment of an allergic disorder is by primary prevention. "Primary prevention" is the effect of preventing or reducing the risk of sensitization of patients to allergens, characterized by absence or reduced levels of allergen-specific IgE antibodies. Preventing or reducing sensitization will result in absence or reduction of allergic symptoms upon exposure to the same allergen. By modulating the way a patient gets sensitized in regard to one allergen or one group of allergens (primary prevention), the subsequent allergic response may also be modulated.

Food allergens are among the first allergens that infants encounter in their early life: typically, cow's milk proteins may be encountered by infants not receiving exclusive breast-feeding. Milk-proteins are indeed among the most frequently observed causes for food allergy in infancy, followed by eggs, soy and wheat proteins. In general, food allergies can manifest in cutaneous (rash, eczema, others) and gastrointestinal symptoms (abdominal cramps; pain, especially in the abdomen; vomiting) in infants and young children. Food allergies are the most common trigger of severe allergic reactions, which may lead to life-threatening anaphylaxis.

Further sensitization and episodes of allergies can also appear when the infant/young child is exposed to a novel food such as cereals, vegetables, fruits, nuts or fish, and also to air-borne allergens such as pollen, house dust mites and animal dander. Adults are affected to a large extent by contact and respiratory allergies. Recent data from the WHO (Clark, M. J. and. Million, R. P (2009) Allergic rhinitis: market evolution, Nature Reviews, Drug Discovery, 8, p.271 -272) indicates that up to 30-40% of the world's population suffer from some form of respiratory allergy.

Animals, particularly small animals such as pets - and especially companion animals such as dogs and cats, may also suffer from food allergies and food intolerances, as well as environmental allergens. These typically manifest in similar symptoms to humans, e.g. gastrointestinal disturbances such as diarrhoea, vomiting and abdominal discomfort, and also dermatitis or pruritis. In small animals, particularly dogs, the most frequent cause of chronic diarrhoea is food-responsive enteropathy (diet-responsive enteropathy or food-responsive diarrhoea).

By preventing or reducing the risk of sensitization of subjects to allergens the compounds and compositions of the present invention may be used for preventing or treating food allergies, food intolerances, respiratory allergies and skin allergies.

In some embodiments an allergic response is a specific IgE-associated immune response and/or a T cell-dependent hypersensitive reaction. Thus, in some embodiments reducing or preventing allergies comprises reducing or preventing specific IgE-associated immune responses and/or a T cell-dependent hypersensitive reaction. In some embodiments allergic inflammation is reduced and/or oral tolerance is enhanced.

A "food allergy" as used herein refers to an abnormal immune response to one or more food allergens, typically an IgE reaction caused by the release of histamine but also encompassing non-IgE immune responses. Symptoms of food allergy may include itchiness, eczema, urticaria, swelling of the tongue, vomiting, diarrhea, hives, trouble breathing, or low blood pressure. When the symptoms are severe and involve more than one system of the body, it is known as anaphylaxis.

As used herein, the term "food allergen" refers to proteins or derivatives thereof that cause abnormal immune responses. Purified food allergens may be named using the systematic nomenclature of the Allergen Nomenclature Sub-Committee of the World Health Organization and International Union of Immunological Societies. Allergen names are composed of an abbreviation of the scientific name of its source (genus: 3-4 letters; species: 1-2 letters) and an Arabic numeral, for example Der p 1 for the first allergen to be described from the house dust mite Dermatophagoides pteronyssinus. Food allergens are derived from proteins with a variety of biologic functions, including proteases, ligand-binding proteins, structural proteins, pathogenesis-related proteins, lipid transfer proteins, profilins, and calcium-binding proteins. A list of food allergens is provided on the official website of the WHO/IUIS Allergen Nomenclature Database, http://www.allergen.org/index.php. (Radauer, C., et al., 2014. Allergy, 69(4), pp.413-419 and Pomés, A., et al., 2018. Molecular immunology).

In one embodiment the food allergen is selected from one or more of the list consisting of: nut, tree nut, peanut, fish, shellfish, molluscs, crustaceans, milk, egg, soy, gluten, cereals, wheat, oats, barley, rye, celery, corn, lupin, sulphites, sesame, mustard, rice, poultry and meat.

A "food intolerance" as used herein refers to a detrimental reaction, often delayed, to a food, beverage, food additive, or compound found in foods that produces symptoms in one or more body organs and systems, but generally refers to reactions other than food allergy. "Food hypersensitivity" may to refer broadly to both food intolerances and food allergies.

A "respiratory allergy" as used herein refers to an abnormal immune response to one or more airborne allergens. Airborne allergens may include pollen, molds or mold spores, weed pollen, tree pollen, grass pollen, and dander. Respiratory allergies may include for example allergic rhinitis and allergic asthma. Symptoms of allergic rhinitis (hay fever) include a runny or stuffy nose, sneezing, red, itchy, and watery eyes, and swelling around the eyes. Symptoms of allergic asthma include episodes of wheezing, coughing, chest tightness, and shortness of breath.

A "skin allergy" as used herein refers to an abnormal immune response caused by contact with one or more environmental allergens or ingestion of allergenic food. Environmental allergens may include a food allergen, dust mite, pollen, molds or mold spores, weed pollen, tree pollen, grass pollen, fleas, pet hair, feathers or pet dander. Skin allergies may include for example dermatitis, atopic dermatitis, contact dermatitis, eczema, atopic eczema, urticaria, and psoriasis. These are typically a group of diseases that results in inflammation of the skin and symptoms include itchiness, red skin and a rash.

Allergic disorders may also include other allergic inflammatory conditions, for example eosinophilic oesophagitis and other eosinophilic-associated gastrointestinal diseases. Eosinophilic esophagitis is an allergic inflammatory condition of the esophagus that involves eosinophils, a type of white blood cell. Symptoms are swallowing difficulty, food impaction, vomiting, and heartburn.

Butyrate, and therefore the compounds and compositions of the present invention may prevent or reduce allergic responses by one or more mechanisms.

The compounds and compositions of the present invention may modulate and/or promote Treg cell differentiation. T regulatory (Treg) cells are critical for tolerance induction and may thus modulate and/or promote oral tolerance induction leading to a prevention from food allergy or leading to a faster outgrowth of food allergy by regulating type 2 allergic response and reducing IgE production. Many chronic inflammatory diseases such as psoriasis, allergies and inflammatory bowel disease are considered to develop via a breakdown in tolerance. Pre-clinical data has demonstrated that direct oral delivery of butyrate increased Treg cell differentiation (Tan, J., et al., 2016. Cell reports, 15(12), pp.2809-2824). Failure to induce Treg activity has been demonstrated to lead to aberrant Th2 responses and the development of allergic disease. Aberrant T helper 2 (Th2) responses may cause allergic inflammation. Type 2 innate lymphoid cells (ILC2s) are a critical source of the Th2 cytokines IL-5 and IL-13, which promote acute asthma exacerbation. It has been demonstrated that butyrate is a critical regulator of ILC2 proliferation and functions through its histone deacetylase (HDAC) inhibitory activity (Thio, C.L.P., et al. 2018. Journal of Allergy and Clinical Immunology, 142(6), pp.1867-1883.e12). Additionnaly, Antigen-specific stimulation of B cells in the presence of butyrate switched plasma cell differentiation to regulatory B cell (Breg) development and IL-10 production (Shi, Y., et al., 2015. Scientific reports, 5, p.17651).

The compounds and compositions of the present invention may increase the production and/or expression of one or more anti-inflammatory cytokines and/or reduce the production and/or expression of one or more pro-inflammatory cytokines and thus reduce tissue inflammation. Butyrate has also been demonstrated to alter the production of anti-inflammatory cytokines e.g. IL-10, (Shi, Y., et al., 2015. Scientific reports, 5, p.17651). Butyrate has also been shown to alter the production of a number of pro-inflammatory cytokines e.g. TNFalpha, IL-5, IL-13, IL-17 (Diakos, C., et al., 2006. Biochemical and biophysical research communications, 349(2), pp.863-868; Thio, C.L.P., et al. 2018. Journal of Allergy and Clinical Immunology, 142(6), pp.1867-1883.e12; and Singh, N., et al., 2014. Immunity, 40(1), pp.128-139.).

The compounds and compositions of the present invention may modulate and/or reduce mast cell activity and all mast cell mediated allergic symptoms such a diarrhoea or hives. Butyrate has been demonstrated to be effective at inhibiting mast cell activation and inflammatory mediator production *in vivo* (Wang, C.C., et al., 2018. Innate immunity, 24(1), pp.40-46).

The compounds and compositions of the present invention may ameliorate GATA-3 expression. GATA-3 is a transcription factor that is specifically expressed in T helper 2 (Th2) cells and plays a critical role in the differentiation of Th2 cells from uncommitted CD4+ lymphocytes. The compound may thus reduce the establishment of the allergic response. In addition, GATA-3 is essential for the gene expression of the cytokines IL-4, IL-5 and IL-13 that mediate allergic inflammation (Barnes, P.J., 2008. Current molecular medicine, 8(5), pp.330-334). A high dose of *in vitro* sodium butyrate (1mM) was sufficient to ameliorate Gata3 expression in Th2 polarized CD4+ T cells with concomitant subversion to IFNγ (Kespohl, M., et al., 2017. Frontiers in immunology, 8, p.1036). 0.1 mM sodium butyrate was insufficient to influence GATA-3 or FoxP3 expression in CD4+ T cells under Th2 polarizing conditions in other studies (Furusawa, Y., et al., 2013. Nature, 504(7480), p.446).

### Administration

Preferably, the compounds and compositions described herein are administered enterally.

Enteral administration may be oral, gastric, and/or rectal.

In one embodiment the administration is oral or gastric. In a preferred embodiment administration is oral.

In general terms, administration of the combination or composition described herein may, for example, be by an oral route or another route into the gastro-intestinal tract, for example the administration may be by tube feeding.

The subject may be a mammal such as a human, canine, feline, equine, caprine, bovine, ovine, porcine, cervine and primates. Preferably the subject is a human.

### Organoleptic properties

The present invention provides compounds that are a source of butyrate having improved organoleptic properties. In particular, the compounds have improved odor and/or taste relative to butyric acid, butyrate salts and/or tributyrin. In one embodiment, the compounds have improved taste relative to tributyrin. In one embodiment, the compounds have improved smell relative to butyrate salts (e.g. sodium butyrate).

In one embodiment, the improved organoleptic properties are improved odour. In one embodiment, the improved organoleptic properties are improved taste. In one embodiment, the improved organoleptic properties are improved odour and improved taste. In one embodiment, the improved taste is reduced bitterness.

### EXAMPLES

### Example 1 - Preparation of butyrated triglycerides (TAG)

Compositions comprising butyrated TAG were generated by chemical interesterification between tributyrin and high oleic sunflower oil in the presence of catalyst such as sodium methoxyde. A molar excess of tributyrin compared to high oleic sunflower oil was be used.

The three reagents, tributyrin, high oleic sunflower oil and the catalyst were mixed together into a reactor under nitrogen atmosphere and then heat under stirring at 80°C for 3h. Once the reaction is completed, the product was washed several times with water then dried under vacuum (25 mBar at 60°C for 2h). The resulting oil product was then subjected to a decoloration step with the action of bleaching earth and was purified either by short-path distillation (130°C, 0.001-0.003 mbar) or by deodorisation (160°C, 2 mbar, 2h) with injection of steam water.

The constituents, mostly triglycerides, of the resulting oil compositions are shown below in Table 1. These triglycerides are represented by the three fatty acids they contain. These fatty acids are represented by their lipid number: 4:0 for butyrate, 16:0 for palmitate, 18:0 for stearate, 18:1 for oleate and 18:2 for linoleate. The fatty acid in the middle is located on the position sn-2 in the triglyceride. As an example, 16:0-4:0-18:1 stands for two different triglycerides having both a butyrate in position sn-2 and either a palmitate in position sn-1 and an oleate in position sn-3 or an oleate in position sn-1 and a palmitate in position sn-3.

TAG profile and regioisomers were analyzed by liquid chromatography coupled to high resolution mass spectrometer. Lipid classes' proportion was evaluated by liquid chromatography coupled to evaporative light scattering detector (ELSD).

**Table 1. TAG regioisomer profile [g/100 g]**

| TAG reg ioisomer *[g*/*100 g]* | |
|---|---|
| | **Composition** |
| 4:0-4:0-4:0 | <0.4-4.7 |
| 4:0-16:0-4:0 | 0.8-1.0 |
| 4:0-18:2-4:0 | 4.0-6.3 |
| 4:0-4:0-18:1 | 3.0-6.1 |
| 4:0-18:1-4:0 | 16.2-27.0 |
| 4:0-18:0-4:0 | 0.8-1.3 |
| 4:0-22:0-4:0 | ≤0.4 |
| 4:0-16:0-18:1 | 1.1-1.5 |
| 16:0-4:0-18:1 | 0.5-0.7 |
| 4:0-18:1-16:0 | 1.2-1.6 |
| 4:0-18:1-18:2 | 2.6-3.1 |
| 18:1-4:0-18:2 | 1.1-1.6 |
| 4:0-18:2-18:1 | 2.9-3.6 |
| 18:1-18:1-4:0 | 23.3-25.8 |
| 18:1-4:0-18:1 | 3.3-4.8 |
| 4:0-18:0-18:1 | 0.9-1.3 |
| 4:0-18:1-18:0 | 0.8-1.1 |
| 4:0-22:0-18:1 | <0.4-0.5 |
| 18:1-18:1-16:0 | 0.6-1.4 |
| 18:1-18:1-18:2 | 1.3-1.5 |
| 18:1-18:2-18:1 | 0.5-0.7 |
| 18:1-18:1-18:1 | 6.1-10.7 |
| 18:1-18:1-18:0 | 0.5-0.8 |
| Total | 93.1-94.1 |

In the Composition samples, the two most abundant TAG are 4:0-18:1-4:0 and 18:1-18:1-4:0, they represent together approximately 40 to 50 g/100 g.

### Example 2 - Odor properties of butyrate moiety containing triglycerides

An odor comparison of a solution including butyrate moiety containing TAG (composed mainly with oleic and butyric fatty acids) was compared to a solution containing sodium butryate.

### Sample preparation

Solutions including butyrate moiety containing TAG (see Example 1) or sodium butyrate were prepared and stored at 4°C prior to delivery to the sensory panel. Each 250 mL solution contained 600 mg of butyric acid (equivalent to one capsule of commercially available sodium butyrate as a supplement; 2.4mg/mL concentration) and 1% w/v BEBA Optipro 1 infant formula in acidified, deionized water.

The samples were prepared the day before the test, by putting 4 mL of each solution (TAG butyrate solution; sodium butyrate solution) in Agilent vials.

### Methodology

The 'two-out-of-five test' was performed. In this test, the panellist is given five samples. The panellist is instructed to identify the two samples that are different from the other three. The presentation order of the samples is randomized in order to avoid presentation order bias.

In addition to the two-out-of-five test, a comment box was presented to the panellists to allow them to comment about the nature of the difference perceived (e.g. odour intensity, odour quality).

### Results

The five samples were presented simultaneously to the panellists. They were asked to uncap, smell and then cap each vial in a given order. The results are shown in Table 2.

**Table 2**

| **Number of responses** | **Number of correct responses** | **Significance** |
|---|---|---|
| **11** | **9** | **p<0.0001***** |

P-value was calculated using a binomial test performed with Fizz software (Biosystemes, France).

The panellists who found the correct responses (butyrate moiety containing TAG different from sodium butyrate) mentioned that the sodium butyrate smells "cheese" whereas for the butyrate moiety containing TAG samples this "cheese" smell was considerably decreased and the odour was quite neutral.

### Example 3 -Taste properties of butyrate moiety containing triglycerides

Sensory benchmarking of a solution including butyrate moiety containing TAG (see Example 1) composed mainly with oleic and butyric fatty acids was performed versus a solution containing tributyrin.

### Sample preparation:

One scoop (4.6g) of BEBA Optipro 1 infant formula was added to warm water (cooled, boiled tap water as per instructions) to a final volume of 150 mL (approximately 3% w/v solution). Each TAG form of butyrate was weighed separately to deliver 600 mg of butyrate, and the addition of infant formula to a final volume of 50 mL for each solution was performed.

Solution A included butyrate moiety containing TAG (see Example 1); and solution B contained tributyrin.

### Methodology

A group of panellists performed a repeated blind-coded tasting.

The samples were prepared just prior to the preliminary bitterness assessment, and each solution was vigorously shaken. Tasting cups labelled A and B were filled at the same time with a small volume of the respective solution.

The two samples were presented simultaneously to the panellists. They were asked to taste the solution in a sip and spit fashion, and rank the perceived bitterness on a scale from 0-10; where 0 is no bitterness perceived and 10 resembles the maximum imaginable bitterness.

### Results

Bitterness of Solution A was ranked by panellists at 4.33 ± 1.52, mean ± SD.

Bitterness of Solution B was ranked by panellists at 8.33 ± 1.52, mean ± SD.

These data show that the butyrate moiety containing TAG composition in infant formula was notably less bitter in taste as compared to tributyrin in infant formula.

### Example 4 - Taste properties 1,3-dibutyryl-2-palmitoylglycerol

### 1,3-dibutyryl-2-palmitoylglycerol (BPB) was synthesized as a single compound using the following synthesis:

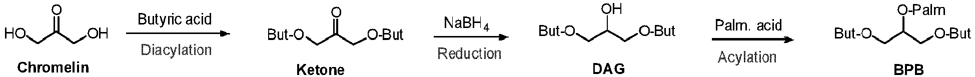

BPB was evaluated in a descriptive sensory panel evaluation and found to be neutral in taste and odor.

### Example 5 - Preparation of butyrated triglycerides (TAG)

Compositions comprising butyrate moiety containing triglycerides were generated by the esterification reaction between monoolein (derived from sunflower oil) with butyric acid added in molar excess (5 equivalents in total). These two reagents were mixed together in a flask and heated to reflux (butyric acid boiling point is 163.5°C). A condenser ("colonne de Vigreux") was used to remove the water. The reaction was monitored by TLC and stopped when all the monoacylglycerol was converted into triacylglycerol.

The constituents, mostly triglycerides, of the resulting oil compositions are shown below in Table 3. As in Example 1, the triglycerides are represented by the three fatty acids they contain. These fatty acids are represented by their lipid number: 4:0 for butyrate, 16:0 for palmitate, 18:0 for stearate 18:1 for oleate and 18:2 for linoleate. The fatty acid in the middle is located on the position sn-2 in the triglyceride.

**Table 3. Trigylceride profile [% by weight]**

| | |
|---|---|
| 4:0-4:0-18:1&4:0-18:1-4:0 | 65.64 |
| 18:1-18:1-4:0& 18:1-4:0-18:1 | 12.53 |
| 4:0-4:0-18:2&4:0-18:2-4:0 | 5.43 |
| 4:0-4:0-18:0&4:0-18:0-4:0 | 3.03 |
| 4:0-18:1-18:2&isomers | 2.98 |
| 4:0-16:0-18:1&isomers | 1.69 |
| 4:0-4:0-16:0&4:0-16:0-4:0 | 1.40 |
| 4:0-4:0-4:0 | 1.36 |
| 4:0-18:0-18:1&isomers | 0.99 |
| 4:0-4:0-22:0&4:0-22:0-4:0 | 0.82 |
| 18:1-18:1-18:1 | 0.63 |
| 4:0-22:0-18:1&isomers | 0.33 |
| 4:0-4:0-24:0&4:0-24:0-4:0 | 0.31 |
| 4:0-4:0-20:0&4:0-20:0-4:0 | 0.28 |
| 4:0-16:0-18:0&isomers | 0.25 |
| 18:0-18:0-16:0 | 0.22 |
| 4:0-16:0-18:2&isomers | 0.21 |
| 4:0-4:0-20:1&4:0-20:1-4:0 | 0.20 |
| 18:1-18:1-18:2 | 0.17 |
| 18:2-18:2-4:0&18:2-4:0-18:2 | 0.17 |
| 18:0-18:0-4:0&18:0-4:0-18:0 | 0.16 |
| 16:0-16:0-4:0&16:0-4:0-16:0 | 0.14 |
| 16:0-18:0-16:0 | 0.12 |
| 4:0-4:0-18:3&4:0-18:3-4:0 | 0.11 |
| 4:0-4:0-16:1&4:0-16:1-4:0 | 0.11 |

In the composition, 4:0-4:0-18:1 was identified as the most abundant triglyceride.

The resulting oil product was then subjected to a decoloration step with the action of bleaching earth and was purified either by short-path distillation (130°C, 0.001-0.003 mbar) and/or by deodorisation (160°C, 2 mbar, 2h) with injection of steam water, to remove residual reagents and intermediates e.g. butyric acid, MAG and byproducts e.g. DAG and tributyrin.

The resulting oil product was evaluated in a descriptive sensory evaluation and found to have a better odour and taste than tributyrin and butyric acid.

### Example 6 - Administration of butyrated triglycerides (TAG) in infant formula and decrease in allergic response in mammals with pre-existing allergy

An allergy to milk was induced in female BALB/c mice aged eight weeks by sensitization via epicutaneous application of the milk protein β-lactoglobulin (BLG) ("Allergic group"). At this stage all three "Allergic groups" (Allergic group 2, Allergic group 2 and Allergic group 3) were subjected to the same sensitization via epicutaneous application of the milk protein β-lactoglobulin (BLG) treatment. The non-allergic negative control group received phosphate buffered saline (PBS) only ("Control group"). Three patches were applied in total, each separated by a week without patch in between. Six days following the final skin patch, mice were orally challenged with BLG following a conventional procedure known to those skilled in the art. Serum was processed for ELISA and BLG-specific Immunoglobulin E (IgE) and BLG-specific Immunoglobulin G1 (lgG1) were determined (Figure 1 and 2 respectively). As can be seen from Figures 1 and 2, the mice in all three of the Allergic groups 1, 2 and 3 showed allergic response to the BLG oral challenge, evidence that the mice were sensitized to the milk protein β-lactoglobulin (BLG).

Following the induction of milk allergy, "Control group" and "Allergic group 1" received infant formula for nutritional management of cow's milk allergy (the extensively hydrolyzed infant formula available commercially under the Trademark Althéra ^{®} was used in this example) reconstituted in the drinking water for a three-week period. "Allergic group 2" and "Allergic group 3" received butyrate *ad libitum* in the presence of infant formula for nutritional management of cow's milk allergy reconstituted in the drinking water for a three-week period. Allergic group 2 received butyrate in the form of sodium butyrate and Allergic group 3 received butyrate in the form of buytrated TAG composition prepared according to Example 1 (referred to herein as "palatable butyrate"). Sodium butyrate or palatable butyrate were prepared to a final concentration of 600 µg butyrate per milliliter of infant formula. Subsequently, an allergy to egg was induced in "Allergic group 1"; "Sodium butyrate treated allergic group 2" and "Palatable butyrate treated allergic group 3" via intraperitoneal administration of the egg protein ovalbumin (OVA) with aluminum hydroxide adjuvant. The "Control group" received intraperitoneal administration of adjuvant alone. Two weeks following the final intraperitoneal administration, mice were orally challenged with OVA three times a week for a total of 12 challenges. All groups continued to receive infant formula +/- butyrate throughout the egg allergy exposure. Mice were monitored after each oral OVA challenge to define a clinical score. Allergy symptoms were defined as follows: 0 = normal stools; 1 = soft/sticky stools; 2 = loose stools; 3 = liquid stools/diarrhea; 4 = at least 2 episodes of liquid diarrhea; 5= score 4 at end of the study. These data were combined to generate a cumulative clinical score for each mouse in the study (Figure 3). From Figure 3 it is seen that the "Sodium butyrate treated allergic group 2" and "Palatable butyrate treated allergic group 3" exhibited a reduction of allergic symptoms compared to "Allergic group 1" that did not receive any butyrate treatment. Serum was processed for ELISA and OVA-specific IgE, OVA-specific IgG1 and mast cell protease-1 (MCPT-1) were determined (Figure 4, 5 and 6 respectively). The similar OVA-specific IgE and OVA-specific IgG1 levels observed in the three Allergic groups indicates that the reduced allergic response following oral OVA challenge cannot be attributed to a reduction in allergic sensitization to the allergen. Figure 6 shows a reduction in mast cell protease-1 in the "Sodium butyrate treated allergic group 2" and "Palatable butyrate treated allergic group 3", indicating modulation of mast cell response in the butyrate treated groups.

This example demonstrates that oral administration of butyrate in mammals with pre-existing milk protein allergy reduces the allergic response to egg allergen.

## Claims

1. A compound having the formula 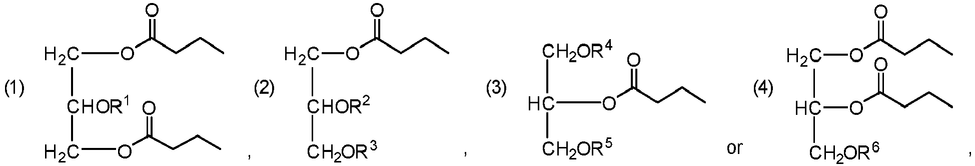 or combinations thereof, for use in the treatment or prevention of an allergic disorder, wherein R¹, R², R³, R⁴, R⁵ and R⁶ are independently, a long chain fatty acid having between 16 and 20 carbons.

2. A composition comprising a compound having the formula 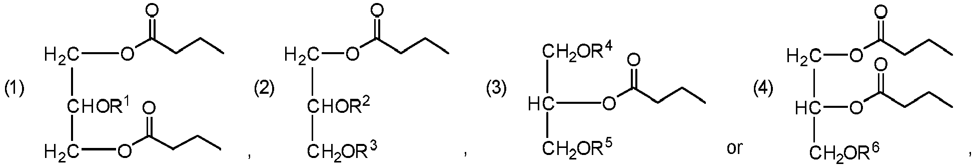 or combinations thereof, for use in the treatment or prevention of an allergic disorder, wherein R¹, R², R³, R⁴, R⁵ and R⁶ are independently, a long chain fatty acid having between 16 and 20 carbons.

3. A composition for use according to claim 2, wherein the composition comprises the compound having formula (1), the compound having formula (2), the compound having formula (3) and the compound having formula (4).

4. A composition for use according to claim 2 or claim 3, wherein the compounds having formula (1), (2), (3) and (4), comprise at least 50%, 60%, 70%, 80%, 90%, 95% or 99% by weight of the total triglycerides of the composition.

5. A composition for use according to any one of claims 2-4, wherein the compounds having formula (1), (2), (3) and (4), comprise at least 50%, 60%, 70%, 80%, 90%, 95% or 99% by weight of the total butyrate moiety containing triglycerides in the composition.

6. A composition for use according to any one of claims 2-5, wherein tributyrin comprises less than 10% by weight of the total triglycerides in the composition, preferably less than 8% by weight, more preferably less than 5% by weight of the total triglycerides in the composition.

7. A composition for use according to any one of claims 2-6, wherein the composition further comprises vitamin A and/or dietary fiber.

8. A composition for use according to any one of claims 2-7, wherein the composition is a nutritional composition, preferably wherein the nutritional composition is a dietary supplement, an infant formula, a follow on-formula, a beverage or a pet care product.

9. A compound or combinations thereof for use according to claim 1, or a composition for use according to any one of any one of claims 2-8, wherein R¹, R², R³, R⁴, R⁵ and/or R⁶ is an unsaturated fatty acid, preferably monounsaturated.

10. A compound or combinations thereof for use according to claim 1 or claim 9, or a composition for use according to any one of any one of claims 2-9, wherein R¹, R², R³, R⁴, R⁵ and/or R⁶ is selected from the group consisting of oleic acid, palmitic acid, or linoleic acid, preferably wherein each of R¹, R², R³, R⁴, R⁵ and R⁶ is oleic acid.

11. A compound or combinations thereof for use according to any one of claims 1, 9 or 10, or a composition for use according to any one of any one of claims 2-10, wherein the allergic disorder is selected from one or more of the group consisting of: a food allergy, a food intolerance, a respiratory allergy and a skin allergy, and/or wherein the allergic disorder is selected from one or more of the group consisting of: rhinitis, asthma, hives, hay fever, allergic conjunctivitis, dermatitis, atopic dermatitis, contact dermatitis, eczema, atopic eczema, urticaria, psoriasis, eosinophilic oesophagitis and other eosinophilic-associated gastrointestinal disease, allergic diarrhea, vomiting, abdominal pain or bloating, and/or wherein the allergen in the allergic disorder is selected from one or more of: a food allergen, dust mite, pollen, molds or mold spores, weed pollen, tree pollen, grass pollen, fleas, pet hair, feathers or pet dander.

12. A compound or combinations thereof for use according to any one of claims 1 or 9-11, or a composition for use according to any one of any one of claims 2-11, wherein the allergen in the allergic disorder is a food allergen, preferably wherein the food allergen is selected from: a nut, tree nut, peanut, fish, shellfish, molluscs, crustaceans, milk, egg, soy, gluten, cereals, wheat, oats, barley, rye, celery, corn, lupin, sulphites, sesame, mustard, rice, poultry and meat.

13. A compound or combinations thereof for use according to any one of claims 1 or 8-12, or a composition for use according to any one of any one of claims 2-14, wherein the compounds or combinations thereof have improved organoleptic properties relative to butyric acid, tributyrin and/or butyrate salts.

## Patentansprüche

1. Verbindung, die die Verbindung aufweist 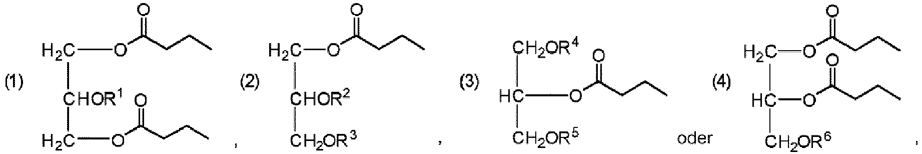 oder Kombinationen davon zur Verwendung bei der Behandlung einer allergischen Erkrankung wobei R¹, R², R³, R⁴, R⁵ und R⁶ unabhängig eine langkettige Fettsäure sind, die zwischen 16 und 20 Kohlenstoffen aufweist.

2. Zusammensetzung, umfassend eine Verbindung, die die Formel aufweist 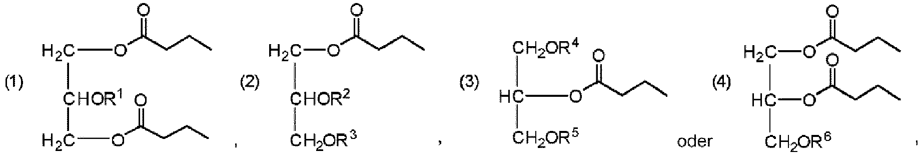 oder Kombinationen davon zur Verwendung bei der Behandlung einer allergischen Erkrankung wobei R¹, R², R³, R⁴, R⁵ und R⁶ unabhängig eine langkettige Fettsäure sind, die zwischen 16 und 20 Kohlenstoffen aufweist.

3. Zusammensetzung zur Verwendung nach Anspruch 2, wobei die Zusammensetzung die Verbindung, die die Formel (1) aufweist, die Verbindung, die die Formel (2) aufweist, die Verbindung, die die Formel (3) aufweist und die Verbindung umfasst, die die Formel (4) aufweist.

4. Zusammensetzung zur Verwendung nach Anspruch 2 oder 3, wobei die Verbindungen, die die Formel (1), (2), (3) und (4) aufweisen, mindestens 50 Gew.-%, 60 Gew.-%, 70 Gew.-%, 80 Gew.-%, 90 Gew.-%, 95 Gew.-% oder 99 Gew.-% der gesamten Triglyceride der Zusammensetzung umfassen.

5. Zusammensetzung zur Verwendung nach einem der Ansprüche 2 bis 4, wobei die Verbindungen, die die Formel (1), (2), (3) und (4) aufweisen, mindestens zu 50 Gew.-%, 60 Gew.-%, 70 Gew.-%, 80 Gew.-%, 90 Gew.-%, 95 Gew.-% oder 99 Gew.-% der gesamten Butyrat-Einheit umfassen, die Triglyceride in der Zusammensetzung enthalten.

6. Zusammensetzung zur Verwendung nach einem der Ansprüche 2 bis 5, wobei Tributyrin weniger als 10 Gew.-% der gesamten Triglyceride in der Zusammensetzung, vorzugsweise weniger als 8 Gew.-%, stärker bevorzugt weniger als 5 Gew.-% der gesamten Triglyceride in der Zusammensetzung, umfasst.

7. Zusammensetzung zur Verwendung nach einem der Ansprüche 2 bis 6, wobei die Zusammensetzung ferner Vitamin A und/oder Ballaststoff umfasst.

8. Zusammensetzung zur Verwendung nach einem der Ansprüche 2 bis 7, wobei die Zusammensetzung eine Nährstoffzusammensetzung ist, wobei vorzugsweise die Nährstoffzusammensetzung ein Nahrungsergänzungsmittel, eine Säuglingsformel, eine Folgenahrungsformel, ein Getränk oder ein Haustierpflegeprodukt ist.

9. Verbindung oder Kombinationen davon zur Verwendung nach Anspruch 1 oder eine Zusammensetzung zur Verwendung nach einem der Ansprüche 2 bis 8, wobei R¹, R², R³, R⁴, R⁵ und/oder R⁶ eine ungesättigte Fettsäure, vorzugsweise einfach ungesättigt, sind.

10. Verbindung oder Kombinationen davon zur Verwendung nach Anspruch 1 oder 9 oder eine Zusammensetzung zur Verwendung nach einem der Ansprüche 2 bis 9, wobei R¹, R², R³, R⁴, R⁵ und/oder R⁶ aus der Gruppe ausgewählt sind, bestehend aus Ölsäure, Palmitinsäure oder Linolsäure, vorzugsweise wobei R¹, R², R³, R⁴, R⁵ und/oder R⁶ Ölsäure sind.

11. Verbindung oder Kombinationen davon zur Verwendung nach einem der Ansprüche 1, 9 oder 10 oder eine Zusammensetzung zur Verwendung nach einem der Ansprüche 2 bis 10, wobei die allergische Erkrankung aus einer oder mehreren der Gruppe ausgewählt ist, bestehend aus: einer Nahrungsmittelallergie, einer Nahrungsmittelunverträglichkeit, einer Atemwegsallergie und einer Hautallergie, und/oder wobei die allergische Erkrankung aus einer oder mehreren der Gruppe ausgewählt ist, bestehend aus: Rhinitis, Asthma, Nesselsucht, Heuschnupfen, allergischer Konjunktivitis, Dermatitis, atopischer Dermatitis, Kontaktdermatitis, Ekzem, atopischem Ekzem, Urtikaria, Psoriasis, eosophiler Ösophagitis und einer anderen eosinophilassoziierten gastrointestinaler Erkrankung, allergischer Diarrhoe, Erbrechen, Bauchschmerzen und Blähungen und/oder wobei das Allergen in der allergischen Erkrankung aus einem oder mehreren ausgewählt ist von: Nahrungsmittelallergen, Hausstaubmilbe, Pollen, Schimmelpilzen oder Schimmelpilzsporen, Kräuterpollen, Baumpollen, Graspollen, Flöhen, Haustierhaar, Federn oder Haustierschuppen.

12. Verbindung oder Kombinationen davon zur Verwendung nach einem der Ansprüche 1 oder 9 bis 11 oder eine Zusammensetzung zur Verwendung nach einem der Ansprüche 2 bis 11, wobei das Allergen in der allergischen Erkrankung ein Nahrungsmittelallergen ist, wobei das Nahrungsmittelallergen vorzugsweise ausgewählt ist aus: einer Nuss, Baumnuss, Erdnuss, Fisch, Schalentieren, Weichtieren, Krustentieren, Milch, Ei, Soja, Gluten, Getreide, Weizen, Hafer, Gerste, Roggen, Sellerie, Mais, Lupine, Sulfiten, Sesam, Senf, Reis, Geflügel und Fleisch.

13. Verbindung oder Kombinationen davon zur Verwendung nach einem der Ansprüche 1 oder 8 bis 12 oder eine Zusammensetzung zur Verwendung nach einem der Ansprüche 2 bis 14, wobei die Verbindungen oder Kombinationen davon verbesserte organoleptische Eigenschaften relativ zu Butansäure, Tributyrin und/oder Butyratsalzen aufweisen.

## Revendications

1. Composé ayant la formule 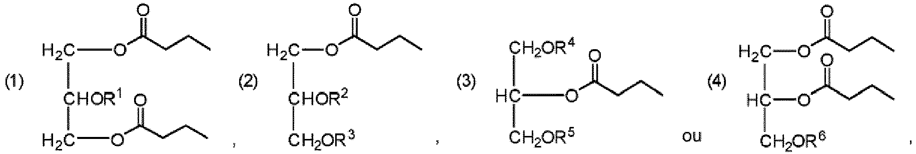 ou combinaisons de celles-ci, pour une utilisation dans le traitement ou la prévention d'un trouble allergique, où R¹, R², R³, R⁴, R⁵ et R⁶ sont indépendamment un acide gras à longue chaîne ayant entre 16 et 20 carbones.

2. Composition comprenant un composé ayant la formule 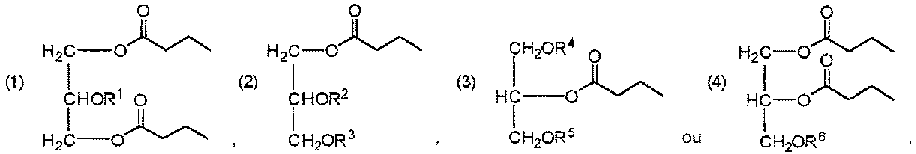 ou combinaisons de celles-ci, pour une utilisation dans le traitement ou la prévention d'un trouble allergique, où R¹, R², R³, R⁴, R⁵ et R⁶ sont indépendamment un acide gras à longue chaîne ayant entre 16 et 20 carbones.

3. Composition pour une utilisation selon la revendication 2, dans laquelle la composition comprend le composé de formule (1), le composé de formule (2), le composé de formule (3) et le composé de formule (4).

4. Composition pour une utilisation selon la revendication 2 ou la revendication 3, dans laquelle les composés de formule (1), (2), (3) et (4), comprennent au moins 50 %, 60 %, 70 %, 80 %, 90 %, 95 % ou 99 % en poids des triglycérides totaux de la composition.

5. Composition pour une utilisation selon l'une quelconque des revendications 2 à 4, dans laquelle les composés de formule (1), (2), (3) et (4), comprennent au moins 50 %, 60 %, 70 %, 80 %, 90 %, 95 % ou 99 % en poids de la fraction butyrate totale contenant des triglycérides dans la composition.

6. Composition pour une utilisation selon l'une quelconque des revendications 2 à 5, dans laquelle la tributyrine comprend moins de 10 % en poids des triglycérides totaux dans la composition, de préférence moins de 8 % en poids, plus préférablement moins de 5 % en poids des triglycérides totaux dans la composition.

7. Composition pour une utilisation selon l'une quelconque des revendications 2 à 6, dans laquelle la composition comprend en outre de la vitamine A et/ou des fibres alimentaires.

8. Composition pour une utilisation selon l'une quelconque des revendications 2 à 7, dans laquelle la composition est une composition nutritionnelle, de préférence la composition nutritionnelle est un complément alimentaire, une préparation pour nourrissons, une préparation de suite, une boisson ou un produit de soin pour animaux de compagnie.

9. Composé ou combinaisons de celui-ci pour une utilisation selon la revendication 1, ou composition pour une utilisation selon l'une quelconque des revendications 2 à 8, où R¹, R², R³, R⁴, R⁵ et/ou R⁶ sont des acides gras insaturés, de préférence mono-insaturés.

10. Composé ou combinaisons de celui-ci pour une utilisation selon la revendication 1 ou la revendication 9, ou composition pour une utilisation selon l'une quelconque des revendications 2 à 9, où R¹, R², R³, R⁴, R⁵ et/ou R⁶ sont choisis dans le groupe constitué de l'acide oléique, l'acide palmitique et l'acide linoléique, de préférence où chacun de R¹, R², R³, R⁴, R⁵et R⁶ est de l'acide oléique.

11. Composé ou combinaisons de celui-ci pour une utilisation selon l'une quelconque des revendications 1, 9 ou 10, ou composition pour une utilisation selon l'une quelconque des revendications 2 à 10, le trouble allergique étant choisi parmi un ou plusieurs du groupe constitué par : une allergie alimentaire, une intolérance alimentaire, une allergie respiratoire et une allergie cutanée, et/ou le trouble allergique étant choisi parmi un ou plusieurs du groupe constitué par les troubles suivants : rhinite, asthme, dermatose inflammatoire, rhume des foins, conjonctivite allergique, dermatite, dermatite atopique, dermatite de contact, eczéma, eczéma atopique, urticaire, psoriasis, oesophagite à éosinophile et autres maladies gastro-intestinale associées à l'éosinophile, diarrhée allergique, vomissements, douleur ou gonflement abdominal(e), et/ou l'allergène du trouble allergique étant choisi parmi un ou plusieurs des éléments suivants : allergène alimentaire, acariens de la poussière, pollen, moisissures ou spores de moisissure, pollen de plantes, pollen d'arbres, pollen d'herbe, puces, poils d'animaux de compagnie, plumes ou squames d'animaux de compagnie.

12. Composé ou combinaisons de celui-ci pour une utilisation selon l'une quelconque des revendications 1 ou 9 à 11, ou composition pour une utilisation selon l'une quelconque des revendications 2 à 11, l'allergène du trouble allergique étant un allergène alimentaire, de préférence l'allergène alimentaire étant choisi parmi : les divers types de noix, l'arachide, le poisson, les fruits de mer, les mollusques, les crustacés, le lait, les oeufs, le soja, le gluten, les céréales, le blé, l'avoine, l'orge, le seigle, le céleri, le maïs, le lupin, les sulfites, le sésame, la moutarde, le riz, la volaille et la viande.

13. Composé ou combinaisons de celui-ci pour une utilisation selon l'une quelconque des revendications 1 ou 8 à 12, ou composition pour une utilisation selon l'une quelconque des revendications 2 à 14, les composés ou leurs combinaisons ayant des propriétés organoleptiques améliorées par rapport à l'acide butyrique, la tributyrine et/ou aux sels de butyrate.
